Europäisches Patentamt

(19) European Patent Office

Office européen des brevets

(11) Veröffentlichungsnummer: **0 013 864**
**B1**

(12) **EUROPÄISCHE PATENTSCHRIFT**

(45) Veröffentlichungstag der Patentschrift:
19.10.83

(51) Int. Cl.³: **A 61 F 1/03**

(21) Anmeldenummer: **79810010.3**

(22) Anmeldetag: **26.01.79**

(54) **Schlittenprothese für das Kniegelenk.**

(43) Veröffentlichungstag der Anmeldung:
**06.08.80 Patentblatt 80/16**

(45) Bekanntmachung des Hinweises auf die Patenterteilung:
**19.10.83 Patentblatt 83/42**

(84) Benannte Vertragsstaaten:
**CH DE FR GB IT NL SE**

(56) Entgegenhaltungen:
**FR-A-2 216 981**
**FR-A-2 269 918**
**FR-A-2 330 377**
**FR-A-2 338 690**
**FR-A-2 339 388**
**FR-A-2 350 825**
**US-A-3 958 278**
**US-A-4 094 017**

(73) Patentinhaber: **OSTEO AG, Bielstrasse 620,
CH-2545 Selzach (CH)**

(72) Erfinder: **Mittelmeier, Heinz, Prof. Dr. med., An der
Schutzhütte, D-Blieskastel (DE)**
Erfinder: **Moser, Heinz, Sparetweg 440, CH-2545 Selzach
(CH)**
Erfinder: **Leu, Beat, Hintere Gasse 33, CH-2500 Biel (CH)**

(74) Vertreter: **Seehof, Michel et al, c/o AMMANN
PATENTANWAELTE AG BERN Schwarztorstrasse 31,
CH-3001 Bern (CH)**

## Schlittenprothese für das Kniegelenk

Die vorliegende Erfindung bezieht sich auf eine Schlittenprothese für das Kniegelenk, mit Prothesenteilen für den Femur, die Tibia und die Patella, die mit mindestens je zwei großen Verankerungszapfen versehen sind.

Als man begann, schadhafte Kniegelenke zu behandeln, verwendete man zunächst Scharnierprothesen aus Metall, die mit langen Verankerungsstielen, zunächst zementfrei im Oberschenkelknochen und im Schienbein verankert wurden. Wegen Lockerungserscheinungen wurden derartige Prothesen dann insbesondere für die zusätzliche Befestigung mit Knochenzement empfohlen. Hier stellten sich jedoch vielfach aseptische Lockerungen an der Zementschicht ein, außerdem bestehen Langzeitbedenken bezüglich des Einsatzes von Knochenzement, insbesondere bei jüngeren Menschen. Der hohe Metallabrieb im Bereich der Scharnierachsen verursacht zudem eine Gewebeschädigung durch das Metallpulver mit erhöhter Metallionenabgabe. Verbesserungen durch Polyäthylengleitlager im Bereich der Achsen oder der Gelenkfläche vermochten das Problem nur unzureichend zu lösen. Insbesondere erschien im Falle des Versagens die für diesen Gelenkersatz notwendige weitgehende Condylenresektion und Aushöhlung des Markraumes ungünstig, da bei Entfernung dieser Prothesen und Rückzug auf eine Arthrodese, das heißt eine Gelenkversteifung, eine ziemliche Beinverkürzung in Kauf zu nehmen war.

Es hat sich deshalb in zunehmendem Maße der Oberflächenersatz des Kniegelenkes in Form sogenannter Schlitten-Prothesen durchgesetzt, wozu nur eine geringfügige oberflächliche Knochenresektion erforderlich ist, so daß im Falle des Versagens noch die Rückzugsmöglichkeit zu einer Scharnier-Prothese oder einer Gelenkversteifung ohne stärkere Verkürzung gegeben ist.

Eine eingangs erwähnte Schlittenprothese ist aus der FR-A 22 69 918 bekannt, die jedoch für die Befestigung mit einem Knochenzement vorgesehen ist, mit allen Nachteilen, die der Knochenzement nach sich zieht. Die Verankerungszapfen sind ausgebildet, um eine gute Haftfähigkeit des Knochenzementes zu erzielen, jedoch nicht, um eine gute direkte Verankerung im Knochen zu erzielen und ein sofortiges unverrückbares Festhalten zu ermöglichen.

Aus der FR-A 22 16 981 ist bekannt, Hüftgelenk- und Ellbogenprothesen zementfrei einzusetzen. Es ist jedoch daraus nicht zu entnehmen, wie eine Schlittenprothese für das Kniegelenk auszustatten ist, um zementfrei eingesetzt zu werden.

Außerdem gibt es eine weitere Anzahl von Schlittenprothesen, die im Femurteil vor allem aus metallischen, den Oberschenkelrollen angepaßten Kufen und entsprechenden Schienbeinkopfflächen aus Polyäthylen bestehen, welche für eine der beiden Gelenkhälften getrennt oder für beide Gelenkhälften unabhängig voneinander oder aber in Verbindung miteinander eingesetzt werden können. Bei Verbindung der Femurteile wird insbesondere auch noch ein Gleitlager für die Kniescheibe mitausgebildet. Ergänzend wurde auch noch ein Gelenkflächenersatz für die Kniescheibengleitfläche aus Polyäthylen geschaffen.

Derartig gestaltete, mit Knochenzement zu verankernde Schlitten-Prothesen weisen jedoch verschiedene Nachteile auf, so eine Verformung des Kunststoffes, einen relativ hohen Kunststoffverschleiß sowie die bereits erwähnte Gefahr der Zerbröckelung der relativ dünnen Zementschicht und damit eine aseptische Prothesenlockerung und bei jüngeren Menschen insbesondere auch die aufgeführten onkologischen Langzeitbedenken gegen den Knochenzement. Insbesondere wurden gehäuft Lockerungen der Tibiakomponenten festgestellt.

Es ist demgegenüber das Ziel der vorliegenden Erfindung, eine Schlitten-Prothese für das Kniegelenk zu schaffen, die ohne Knochenzement befestigt werden kann und insbesondere auch langfristig verankert bleibt und die an den Gelenkflächen einen wesentlich geringeren Verschleiß und einen besseren Reibungswert aufweist als die oben erwähnten vorbekannten Schlitten-Prothesen.

Dieses Ziel wird durch die im kennzeichnenden Teil des Anspruches 1 angegebenen Merkmale erreicht.

Die Erfindung wird nun im einzelnen anhand einer Zeichnung von Ausführungsbeispielen näher erläutert werden.

Figur 1 zeigt die schematische Vorderansicht eines Kniegelenkes,

Figur 2 in vergrößertem Maßstab und im Schnitt den Prothesenteil für das Schienbein,

Figur 3 die Prothese von Figur 2 von unten,

Figur 4 eine schematische Seitenansicht eines Kniegelenkes,

Figur 5 in vergrößertem Maßstab und im Längsschnitt ein Prothesenteil für die Schenkelrolle und

Figur 6 im Querschnitt die Prothese von Figur 5.

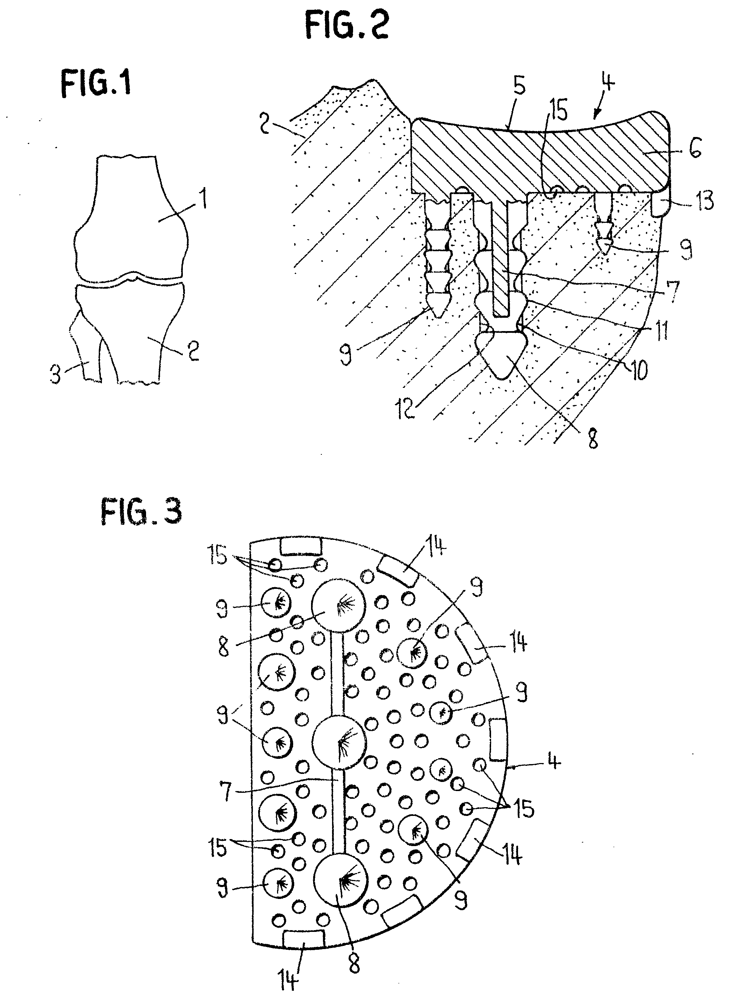

In Figur 1 erkennt man schematisch die beiden Knochenkomponenten des Kniegelenkes, den Femur 1 oder Oberschenkelknochen, die Tibia 2 oder das Schienbein und das Wadenbein 3.

In Figur 2 ist das Prothesenteil 4 für das Schienbein 2, im folgenden kurz Tibiateil genannt, im Schnitt dargestellt. Die Gelenkfläche 5 ist entsprechend der natürlichen Schienbeingelenkfläche leicht konkav, während die Dicke des Plateaus 6 zum Ausgleich von Condyleneffekten verschieden sein kann. In vorliegendem Beispiel weist das Tibiateil einen in der Längsachse des Prothesenteils verlaufenden Verankerungsflügel 7 auf, der mit drei relativ dicken Verankerungszapfen 8 versehen ist. Der Tibiateil weist ferner entlang des Randes eine Anzahl Verankerungszapfen 9 mit unterschiedlichem Durchmesser auf. Sämtliche Verankerungszapfen 8 und 9 weisen im vorliegenden Fall ein sägezahnartiges Profil mit Einschnürungen 10 und Ausbuchtungen 11 auf. Anstelle dieses sägezahnartigen Profils kann auch dasjenige einer Knochenschraube oder eines anderen geeigneteren Gewindes verwendet werden. Aus Figur 2 ist ersichtlich, daß der Durchmesser der Bohrungen 12 etwas größer als der Durchmesser der Einschnürungen und etwas kleiner als der Durchmesser der Ausbuchtungen ist, wodurch eine sehr gute Verankerung beim Einschlagen dieser Zapfen erzielt wird und der Knochen in die Einschnürungen hineinwachsen kann. Zwecks Verstärkung der Verankerung ist der untere Rand des Plateaus mit einem den natürlichen Condylenrand überlappenden Flansch 13 versehen, wobei dieser Flansch entweder durchgehend oder, wie insbesondere aus Figur 3 hervorgeht, aus einzelnen Zähnen 14 bestehen kann. Dadurch wird ein Schutz und eine Abstützung des natürlichen Condylenrandes bewirkt. Die verbleibende, dem Knochen zugewandte Oberfläche weist zwecks Erhöhung der Verankerung Höhlungen 15 in Stecknadelkopfgröße auf, die in einer wabenartigen Struktur angeordnet sind. Im Falle einer totalen Kniearthrose ist vorgesehen, daß die beiden Tibiateile im vorderen Bereich durch eine Brücke zu einer Einheit verbunden werden können. Dadurch wird ein Oberflächenersatz im medialen und lateralen Condylenbereich sowie am Patellagleitlager ermöglicht.

Als Materialien für die Herstellung dieser Tibiateile kann man gebräuchliche körperverträgliche Metalle oder Metall-Legierungen, beispielsweise geschmiedetes Titan, Chrom-Kobalt-, Chrom-Kobalt-Molybdänlegierungen oder Aluminiumoxydkeramik verwenden. Besonders vorteilhaft erscheint jedoch die Verwendung von kohlenstoffaserverstärktem Kunststoff, beispielsweise Polyäthylen. Durch diese Faserverstärkung erhält der Kunststoff zunächst mehr Festigkeit, so daß plastische Verformungen vermieden werden können. Außerdem kann auch die Reibung und somit auch der Verschleiß herabgesetzt werden. Vor allem kommt es aber auch zu einer Erhöhung des Elastizitätsmoduls, welche bedingt, daß auch die elastische Verformung geringer ist, so daß die Belastungen örtlich nicht so stark auf das Knochenlager durchwirken. Vorteilhafterweise sollten die Kohlenstoffasern derart verteilt sein, daß in der Schicht in der Nähe des Knochens möglichst wenig Fasern sind, um einen Elastizitätsmodul zu erzielen, der vergleichbar demjenigen des Knochens ist, um Relativbewegungen zwischen dem Prothesenteil und dem Knochen zu vermeiden. Dabei können beispielsweise kurze Stapelfasern von wenigen Millimetern Länge im pulverisierten Kunststoff vermischt werden, wobei der Prothesenteil durch anschließendes Thermosintern mit Einpressen in die Gußform hergestellt wird. Es ist aber auch möglich, eine Mischung aus thermoplastischem Kunststoffpulver und entsprechenden Kohlenstoffasern zu verflüssigen und in die Prothesenform zu gießen. Bei Konstruktionsteilen der Prothese, die besonders beansprucht werden, kann man entweder die Kohlenstoffasern in besonderen Richtungen ordnen oder lange Faserverbände oder Fasergewebe verwenden.

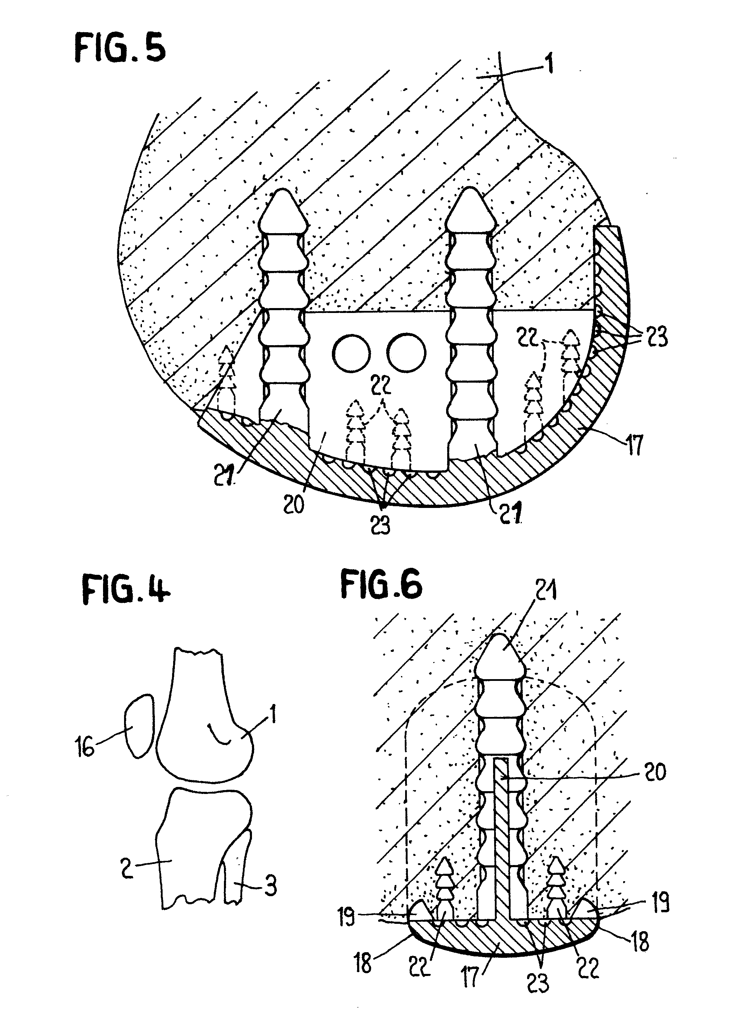

In Figur 4 ist schematisch das Kniegelenk, seitlich gesehen, dargestellt, und man erkennt wieder einen Teil des Femurs 1, der Tibia 2 und des Wadenbeins 3 sowie die Patella, die Kniescheibe 16.

In Figur 5 erkennt man im Schnitt den Prothesenteil für den Femur, ein Femurteil 17 in Form einer Schlittenkufe, die der konvexen Condylenform angepaßt ist. Die Ränder 18 sind in Richtung zum Knochen etwas umgebogen und abgeschrägt und weisen an den Rändern Zähne 19 auf, die eine bessere Verzahnung ermöglichen. Wie das Tibiateil 4 weist auch das Femurteil einen Verankerungsflügel 20, der mit beispielsweise zwei Verankerungszapfen 21 verstärkt ist, sowie weitere kleinere Verankerungszapfen 22 mit unterschiedlichen Durchmessers auf, wobei das Profil der Verankerungszapfen das gleiche ist wie dasjenige der Verankerungszapfen des Tibiateils. Ferner weist die dem Knochen zugewandte Seite des Prothesenteiles Höhlungen 23 auf, die der besseren Verankerung dienen. Entsprechend den Tibiateilen ist auch bei den Femurteilen vorgesehen, daß die Kufen für die mediale und laterale Femurcondyle im vorderen Gelenksbereich derart verbunden sind, daß damit auch ein Ersatz des Kniescheiben-Gleitlagers gewährleistet wird.

Als Material für die Femurteile kommt wie bei den Tibiateilen sowohl körperverträgliche Metalle sowie Metall-Legierungen oder Aluminiumoxydkeramik oder kohlenstoffaserverstärkte Kunststoffe in Frage, wobei verschiedene Paarungen der Femur- und Tibiateile denkbar sind, beispielsweise die Femurteile aus Metall oder Aluminiumoxydkeramik und die Tibiateile aus faserverstärktem Kunststoff oder umgekehrt die Tibiateile aus Metall oder Aluminiumoxydkeramik und die Femurteile aus faserverstärktem Kunststoff oder sämtliche Teile aus Aluminiumoxydkeramik oder sämtliche Teile aus Kohlenstoffaserverstärktem Kunststoff.

Der Oberflächenersatz für die Kniescheibe ist in entsprechender Weise vorgesehen, wobei dessen Gelenkfläche der Oberfläche des Patellagleitlagers der Femurcondyle angepaßt ist und die dem Knochen zugewandte Seite in ähnlicher Weise wie vorgehend beschrieben mit zahlreichen Verankerungsstiften und wabenartig angeordneten Höhlungen zum Einwachsen des Knochens vorgesehen ist.

Eine weitere wesentliche Verbesserung des Anwachsens des Knochens an die Prothese kann durch biologische Oberflächenaktivierung mittels Einlagerung bioaktiver Partikel erzielt werden, wobei insbesondere an apatithaltige Gläser, sogenanntes Bioglas, Apatitpartikel und hetero-homöo- sowie autoplastische enteiweißte Knochenpartikel gedacht ist. Im Falle von Prothesenteilen aus faserverstärktem Kunststoff sollen diese Partikel über-äquatorial in die in Frage kommende Prothesenoberfläche eingeschlossen werden, wobei das zwischengelagerte Kunststoffmaterial praktisch als Dehnungsfuge dienen kann, womit ein Zerbröckeln oder Abplatzen des bioaktiven Materials durch elastische oder plastische Verformung vermieden wird, im Gegensatz zu bisher vorgeschlagenen zusammenhängenden Beschichtungen.

Die Einlagerung der Partikel in die Oberfläche kann beispielsweise dadurch erreicht werden, daß die Partikel zunächst mit einem möglichst wasserlöslichen Klebstoff an den entsprechenden Stellen der Prothesengußform durch Aufschüttung angebracht werden und dann beim Eingießen des erwärmten, flüssigen Kunststoffmaterials mit den Kohlefasern oder durch Hitzepressung feinpulvrigen Kunststoffmaterials mit den Kohlefasern in die Oberfläche eingeschmolzen, beziehungsweise eingepreßt werden.

Die Beschichtung beziehungsweise Einlagerung der bioaktiven Partikel in Metall oder Aluminiumoxydkeramik könnte prinzipiell ähnlich erfolgen, das heißt durch das Aufgießen von Metall auf eine ähnlich beschichtete Gußform oder durch Aufgießen der Aluminiumoxydkeramik-Paste auf die Gußform und nachträgliches Einbrennen. In allen Fällen ist es vorteilhaft, eine nichtzusammenhängende Beschichtung zu erzielen. Im Falle von Aluminiumoxydkeramik werden vorzugsweise Partikel aus apatithaltigen Gläsern verwendet.

Die Einlagerung dieser bioaktiven Partikel in die auf die Knochen liegenden Oberflächen der Prothesen trägt zur weiteren Aufrauhung und somit Vergrößerung der Oberfläche und damit zur Minderung des spezifischen Druckes bei. Dies ermöglicht vor allem — in Verbindung mit der festen, primären, mechanischen Verankerung — ein direktes Anwachsen des Knochengewebes ohne bindegewebige Zwischenschicht und eine physikalisch-chemische Verbindung der Prothese an das Knochengewebe im Sinne einer mikroskopischen Punktverschweißung. Gleichzeitig wird damit die chemische Einwirkung des nichtbioaktiven Prothesenmaterials auf das Knochengewebe und damit die Gefahr der chemischen Fremdkörperreaktion reduziert.

Die Operationstechnik für das Einsetzen der Prothesenteile geht aus der bekannten Operationstechnik für solche Teile hervor. Nach Freilegung und Vorbereitung der entsprechenden Knochenpartien wird eine dem einzusetzenden Prothesenteil entsprechende Schablone angesetzt und der für den Verankerungsflügel notwendige Schlitz gemäß Schablone eingefräst und die Bohrungen für die Verankerungszapfen gebohrt. Im Falle, daß an einem Prothesenteil zwei zueinander senkrecht stehende Verankerungsflügel angeordnet sind, muß eine entsprechende Schablone verwendet werden. Gegebenenfalls ist es vorteilhaft oder notwendig, die Schablone mittels einer Spongiosaschraube zu fixieren, um deren Verschieben während dem Bohren der Löcher zu verhindern. Anschließend wird der Prothesenteil aufgesetzt und mittels eines Spezialinstrumentes eingeschlagen, wobei beispielsweise für das Einschlagen der Tibiateile ein gekrümmtes Instrument verwendet werden muß, da die Oberschenkelrolle ein geradliniges Instrument behindern würde.

## Patentansprüche

1. Schlittenprothese für das Kniegelenk, mit Prothesenteilen (4, 17) für den Femur (1), die Tibia (2) und die Patella (16), die mit mindestens je zwei großen Verankerungszapfen (8, 21) versehen sind, dadurch gekennzeichnet, daß zur zementfreien Befestigung und zum sofortigen verrückungsfreien Festhalten die Prothesenteile zusätzlich mit einer Anzahl in der Nähe des Prothesenteil-Randes angeordneter kleinerer Verankerungszapfen (9, 22) versehen sind und sämtliche Verankerungszapfen ein sägezahnähnliches (10, 11) oder Knochenschrauben-förmiges Profil aufweisen.

2. Schlittenprothese nach Anspruch 1, dadurch gekennzeichnet, daß die auf den Knochen (1, 2) zu liegen kommende Oberfläche der Prothesenteile mit Höhlungen (15, 23) versehen ist.

3. Schlittenprothese nach Anspruch 1 oder 2, dadurch gekennzeichnet, daß das Plateau (6) des für die Tibia (2) bestimmten Prothesenteils (4) mit einem nach unten weisenden Flansch (13) versehen ist.

4. Schlittenprothese nach Anspruch 3, dadurch gekennzeichnet, daß der Flansch (13) aus einer Anzahl Zähnen (14) gebildet ist.

5. Schlittenprothese nach einem der Ansprüche 1 — 4, dadurch gekennzeichnet, daß alle oder einzelne Teile aus kohlenstoffaserverstärktem Kunststoff gefertigt sind.

6. Schlittenprothese nach Anspruch 5, dadurch gekennzeichnet, daß die Kohlenstoffasern derart angeordnet sind und ihr Anteil derart bemessen ist, daß die unterste, an den Knochen angrenzende Schicht einen demjenigen des Knochens vergleichbaren Elastizitätsmodul aufweist.

7. Schlittenprothese nach einem der Ansprüche 1 — 4, dadurch gekennzeichnet, daß alle oder einzelne Teile aus Aluminiumoxydkeramik gefertigt sind.

8. Schlittenprothese nach einem der Ansprüche 1 — 7, dadurch gekennzeichnet, daß die Tibiateile (6) aus kohlenstoffaserverstärktem Kunststoff und die Femurteile (17) aus Metall oder Aluminiumoxydkeramik hergestellt sind, oder umgekehrt und die Patellateile aus kohlenstoffaserverstärktem Kunststoff.

9. Schlittenprothese nach einem der Ansprüche 1 – 8, dadurch gekennzeichnet, daß die mit dem Knochen in Berührung kommenden Oberflächen mit nicht resorbierbaren bioaktiven Partikeln, wie apatithaltige Gläser, Apatit, enteiweißtes Knochenmaterial, beschichtet sind, wobei die Partikel über-äquatorial umfaßt sind und eine nicht-zusammenhängende Schicht bilden.

## Claims

1. Sledge prosthesis for knee-joint with prosthesis parts (4, 17) for the femur (1), the tibia (2) and the kneecap (16), each of said prosthesis parts being provided with at least two great anchoring pins (8, 21), characterized in that for an attachement without cement and an immediate retention without displacement of the prosthesis parts the prosthesis parts comprise moreover a number of smaller anchoring pins (9, 22) arranged in the vicinity of the edge of the prosthesis part, and that all anchoring pins have a saw-tooth like (10, 11) or a bone screw like profile.

2. Sledge prosthesis according to claim 1, wherein the surface of the prosthesis parts lying on the bones (1, 2) is provided with cavities (15, 23).

3. Sledge prosthesis according to claim 1 or 2, wherein the plateau (6) of the prosthesis part intended for the tibia (2) is provided with a flange (13) pointing at the bottom.

4. Sledge prosthesis according to claim 3, wherein said flange (13) is formed by a number of teeth (14).

5. Sledge prosthesis according to one of the claims 1 – 4, wherein all parts or individual parts are manufactured in carbon fibres reinforced plastics material.

6. Sledge prosthesis according to claim 5, wherein the carbone fibers are so arranged and their proportion thereof is determined, so that the lowest layer, adjoining the bone, has an modulus of elasticity comparable to that of the bone.

7. Sledge prosthesis according to one of the claims 1 – 4, wherein all parts or individual parts are manufactured in aluminium oxide ceramics.

8. Sledge prosthesis according to one of the claims 1 – 7, wherein the tibia parts (6) are manufactured in carbone fibre-reinforced plastics material and the femur parts (17) in metal or aluminium oxide ceramics, or conversely, and the kneecap parts in carbon fibres reinforced plastics material.

9. Sledge prosthesis according to one of the claims 1 – 8, wherein the surfaces which come into contact with the bone are covered with bioactive non-resorbable particles such as apatite containing glasses, apatite, dealbumined bone material, the particles being super-equatorially covered and forming a non-coherent layer.

## Revendications

1. Prothèse à glissière pour l'articulation du genou avec des parties de prothèse (4, 17) pour le fémur (1), le tibia (2) et la rotule (16), lesdites parties de prothèse étant pourvues chacune d'au moins deux grandes chevilles (8, 21), caractérisée en ce que pour une fixation sans ciment et une rétention immédiate et sans déplacement des parties de prothèse les parties de prothèse comprennent en outre un nombre de plus petites chevilles (9, 22) disposées au voisinage du bord de la partie de prothèse, et que toutes les chevilles comprennent un profil semblable à une dent-de-scie (10, 11) ou en forme de vis à os.

2. Prothèse à glissière selon la revendication 1, caractérisée en ce que la surface des parties de prothèse reposant sur les os (1, 2) est pourvue de creusures (15, 23).

3. Prothèse à glissière selon la revendication 1 ou 2, caractérisée en ce que le plateau (6) de la partie de prothèse (4) destinée au tibia (2) est pourvu d'une collerette (13) pointant vers le bas.

4. Prothèse à glissière selon la revendication 3, caractérisée en ce que ladite collerette (13) est formée d'un nombre de dents (14).

5. Prothèse à glissière selon l'une des revendications 1 – 4, caractérisée en ce que toutes les parties ou des parties individuelles sont fabriquées en matériau synthétique renforcé par fibres de carbone.

6. Prothèse à glissière selon la revendication 5, caractérisée en ce que la disposition des fibres de carbone et leur quote-part sont telles que la couche la plus basse, contigue à l'os, a un module d'élasticité comparable à celui de l'os.

7. Prothèse à glissière selon l'une des revendications 1 – 4, caractérisée en ce que toutes les parties ou des parties individuelles sont fabriquées en céramique d'oxyde d'aluminium.

8. Prothèse à glissière selon l'une des revendications 1 – 7, caractérisée en ce que les parties de tibia (6) sont fabriquées en matériau synthétique renforcé par fibres de carbone et les parties de fémur (17) en métal ou en céramique d'oxyde d'aluminium, ou vice-versa, et les parties de rotule en matériau synthétique renforcé par fibres de carbone.

9. Prothèse à glissière selon l'une des revendications 1 – 8, caractérisée en ce que les surfaces venant en contact avec l'os sont couvertes de particules bioactives non-résorbables telles que verres contenant de l'apatite, apatite, matériel osseux sans albumine, les particules étant enrobées au-delà de leur équateur et formant une couche discontinue.

# FIG.2

# FIG.1

# FIG.3

# FIG.5

1

22

23

22

17

21

20

23

21

# FIG.4

1

16

2

3

# FIG.6

21

20

19

19

18

18

22

17

22

23